# DEMANDE DE BREVET EUROPEEN

(11) **EP 2 845 558 A1**
(43) Date de publication de la demande: **11.03.2015**
(21) Numéro de dépôt: 14183807.8
(22) Date de dépôt: 05.09.2014
(51) Int. Cl.: A61B 19/08

(54) **Champ d'accouchement multipositions**

(30) Priorité: 06.09.2013 FR 1358592
(71) Demandeur: Vygon, 95440 Ecouen (FR)
(72) Inventeur: Lestoquoy, Patrick, 59551 Attiches (FR)
(74) Mandataire: Regimbeau

(57) **Abrégé**

L'invention concerne un champ d'accouchement (1), comprenant une feuille (2) souple présentant une partie supérieure (3), destinée à recevoir une parturiente, et une partie inférieure (4) s'étendant dans le prolongement de la partie supérieure (3) et fixée de manière amovible sur la partie supérieure (3), la feuille (2) étant réalisée dans un matériau hydrophobe formant barrière aux liquides et la partie inférieure (4) de la feuille (2) comprend une face supérieure (40) comprenant un matériau absorbant.

## Description

### DOMAINE DE L'INVENTION

L'invention concerne d'une façon générale le matériel médical, et en l'espèce un champ d'accouchement, adapté pour être utilisé que ce soit en position latérale ou obstétricale en garantissant une position et un confort optimaux de la parturiente.

### ARRIERE-PLAN TECHNOLOGIQUE

Les champs d'accouchement sont des feuilles souples généralement étanches, qui sont placées sur des tables de travail et d'accouchement afin d'une part de protéger la table et d'autre part d'apporter du confort aux parturientes.

L'accouchement est généralement pratiqué en position dite « obstétricale », dans laquelle la parturiente est allongée sur le dos et a les pieds en position surélevée et écartée par rapport à son bassin. Il a également été proposé une nouvelle position, dite latérale, dans laquelle la parturiente est allongée sur le côté et n'a qu'une seule jambe surélevée par rapport aux hanches, par exemple en appui sur un étrier.

Les tables de travail et d'accouchement actuelles sont de plus en plus sophistiquées et comprennent plusieurs parties mobiles les unes par rapport aux autres, voire également des composants électroniques. Par exemple, les tables de montage actuelles comprennent souvent un dossier, une assise et une partie support, s'étendant dans le prolongement de l'assise et pouvant être abaissée ou escamotée sous celle-ci, selon la position choisie par la parturiente. Le dossier et l'assise de la table sont également mobiles entre eux et permettent le passage de la table dans la position de Trendelenburg, formant un V, qui est très souvent requise au cours des différentes phases du travail ainsi que pendant et après la délivrance.

Il devient donc de plus en plus nécessaire de protéger ces tables des fluides pouvant provenir de l'accouchement, afin d'une part de les préserver et d'autre part de simplifier le travail de nettoyage et de remise au propre par le personnel d'entretien suite à un accouchement.

De nos jours, et quelle que soit la position d'accouchement, la table de travail et d'accouchement est recouverte d'un drap sur lequel la parturiente est installée afin de commencer le travail. Néanmoins, lorsque la table est en position de Trendelenburg, les fluides physiologiques (notamment le liquide amniotique) ont tendance à se concentrer le long de la ligne de cassure entre le dossier et l'assise de la table (fond du V), et sont difficiles à nettoyer en raison de leur volume très important, notamment lors d'une rupture soudaine de la poche des eaux. Afin d'absorber ces fluides, les opérateurs rapportent habituellement des champs absorbants du type Absorbex® sur le drap recouvrant la table. Il s'avère cependant que ces champ absorbants se trouvent souvent soit entrainés par les fluides, soit insuffisamment nombreux ou insuffisamment efficaces pour absorber efficacement les fluides le long de cette ligne de cassure.

Lors de l'expulsion du placenta et le suivi des pertes sanguines, qui se fait majoritairement dans la position gynécologique, un nouveau champ absorbant est alors rapporté sur la table en remplacement éventuellement des champs absorbants précédents, afin de permettre le suivi de ces pertes par l'opérateur. Le cas échéant, un autre champ encore est rapporté sur la table lorsqu'un geste opératoire devant être pratiqué dans des conditions aseptiques stériles, tel qu'une suture d'épisiotomie ou la prise en charge d'une hémorragie postpartum, doit être réalisé, afin de remettre la table au propre.

Les différentes étapes du travail, de la délivrance, du suivi des pertes sanguines et éventuellement de la suture d'épisiotomie nécessitent donc de pouvoir manipuler la table afin de la faire passer dans les différentes positions pour suivre la mobilité de la parturiente sans pour autant la gêner, et de rapporter une pluralité de champs absorbants afin de garantir le confort de la parturiente et de protéger la table.

Le document US 2005/022822 décrit un champ opératoire comprenant une feuille souple présentant une partie supérieure (deuxième feuille) et une partie inférieure (première feuille), s'étendant dans le prolongement de la partie supérieure. La partie supérieure de la feuille comprend en outre une face absorbante. Toutefois, un tel champ peut difficilement être mis en oeuvre dans le cadre d'un accouchement. On rappelle en effet qu'un champ d'accouchement doit être susceptible de recevoir une plus grande quantité de fluides au cours de l'accouchement qu'un champ opératoire conventionnel, en particulier lorsque la table opératoire est placée en position de Trendelenburg, dans la mesure où les fluides ont tendance à s'accumuler au niveau de la ligne de cassure entre le dossier et l'assise. Or, ces fluides ne peuvent en aucun cas être évacués par les champs d'accouchement conventionnels et encore moins par le champ opératoire de ce document puisqu'ils présentent une face absorbante en partie supérieure.

Le document US 4 489 720 concerne quant à lui un champ d'accouchement comprenant une feuille souple présentant une partie supérieure, destinée à recevoir une parturiente, et une partie inférieure, s'étendant dans le prolongement de la partie supérieure. Ce document n'adresse cependant pas la problématique de la protection de la table de travail et d'accouchement.

### RESUME DE L'INVENTION

Un objectif de l'invention est de proposer un champ d'accouchement capable de protéger efficacement une table de travail et d'accouchement durant toutes les étapes de l'accouchement, c'est-à-dire du travail à la mesure des pertes sanguines après la délivrance, quelle que soit la position d'accouchement choisie, sans nécessiter de rapporter des champs absorbants à chacune de ces étapes et qui permette en outre à un opérateur d'effectuer un geste opératoire, notamment une suture d'épisiotomie, dans des conditions aseptiques stériles.

La difficulté est de proposer un champ qui puisse protéger du matériel dans un milieu non stérile, tel qu'une salle d'accouchement, tout en permettant un geste opératoire dans des conditions d'aseptie stérile associé au suivi de l'hémorragie postpartum.

Pour cela, l'invention propose un champ d'accouchement comprenant une feuille souple, ladite feuille souple présentant une partie supérieure, destinée à recevoir une parturiente, et une partie inférieure, s'étendant dans le prolongement de la partie supérieure, dans lequel la partie supérieure comprend une face supérieure qui est destinée à venir en contact avec la parturiente, ladite face supérieure étant réalisée dans un matériau hydrophobe formant barrière aux liquides, et en ce que la partie inférieure de la feuille comprend une face supérieure comprenant un matériau absorbant.

Certaines caractéristiques préférées mais non limitative du champ d'accouchement décrit ci-dessus sont les suivantes :
- la partie inférieure de la feuille est fixée de manière amovible sur la partie supérieure,
- la partie inférieure est fixée sur un bord inférieur de la partie supérieure par l'intermédiaire d'un adhésif approprié ou par thermoscellage d'une bande intermédiaire formée d'un multicouche pelable, ou est reliée à la partie supérieure par l'intermédiaire d'une ligne de faiblesse,
- le champ d'accouchement comprend en outre une poche de recueil, fixée sur la feuille de manière à s'étendre sous la partie inférieure et à être protégée par ladite partie inférieure au cours de l'accouchement,
- la poche de recueil est fixée dans une portion inférieure de la partie supérieure de la feuille supérieure,
- le champ d'accouchement comprend en outre un pagne logé dans la poche de recueil, ledit pagne pouvant être fixé sur une face interne de ladite poche de recueil,
- la feuille est réalisée dans un matériau hydrophobe formant barrière aux liquides du type polyéthylène,
- le matériau absorbant de la face supérieure (40) de la partie inférieure (4) est fixé sur ladite feuille une face supérieure de la partie supérieure, adaptée pour venir en contact d'une parturiente, est formée dans un matériau hydrophobe formant barrière aux liquides,
- la face supérieure de la partie supérieure comprend un nontissé hydrophobe,
- le champ d'accouchement est adapté pour être placé sur une table de travail et d'accouchement, et la feuille comprend en outre des moyens de fixation à ladite table d'accouchement,
- les moyens de fixation comprennent un rabat, formé dans une partie supérieure de la feuille, adapté pour être enfilé sur un bord supérieur de la table de travail et d'accouchement,
- le champ d'accouchement comprend en outre au moins une poche latérale, fixée sur un bord latéral de la partie supérieure de la feuille, et adaptée pour recueillir des fluides lors de l'accouchement,
- le champ d'accouchement comprend deux poches latérales, s'étendant en regard et fixées sur deux bords latéraux opposés de la partie supérieure de la feuille, et
- la feuille présente une longueur comprise entre deux mètres et trois mètres.

Selon un deuxième aspect, l'invention propose également un champ d'accouchement, adapté pour protéger une table de travail et d'accouchement, comprenant une feuille souple, ladite feuille souple présentant une partie supérieure destinée à recevoir une parturiente, dans lequel la partie supérieure de la feuille comprend des moyens de fixation à la table de travail et d'accouchement.

Certaines caractéristiques préférées mais non limitatives du champ d'accouchement selon ce troisième aspect sont les suivantes :
- les moyens de fixation comprennent un rabat, formé dans une partie supérieure de la feuille, adapté pour être enfilé sur un bord supérieur de la table de travail et d'accouchement,
- le rabat comprend un bord supérieur de la feuille supérieur replié sur lui-même et fixé dans cette position, par exemple par soudage ou à l'aide d'un adhésif,
- la feuille présente une longueur comprise entre deux mètres et trois mètres,
- le champ opératoire comprend en outre une partie inférieure, s'étendant dans le prolongement de la partie supérieure, ladite partie inférieure étant fixée de manière amovible sur la partie supérieure,
- la partie inférieure est fixée sur un bord inférieur de la partie supérieure par l'intermédiaire d'un adhésif approprié ou par thermoscellage d'une bande intermédiaire formée d'un multicouche pelable, ou est reliée à la partie supérieure par l'intermédiaire d'une ligne de faiblesse.
- le champ opératoire comprend en outre une poche de recueil, fixée sur la feuille, par exemple dans une portion inférieure de la partie supérieure de la feuille, de manière à s'étendre sous la partie inférieure,
- la poche de recueil est amovible,
- le champ comprend en outre un pagne, logé dans la poche de recueil, qui peut par exemple être fixé sur une face interne de celle-ci,
- la feuille est réalisée dans un matériau hydrophobe formant barrière aux liquides du type polyéthylène,
- la partie inférieure de la feuille comprenant une face supérieure comprenant un matériau absorbant,
- une face supérieure de la partie supérieure, adaptée pour venir en contact avec une parturiente, est comprend un matériau hydrophobe formant barrière aux liquides,
- la face supérieure de la partie supérieure comprend un nontissé hydrophobe, et
- le champ comprend en outre au moins une poche latérale, fixée sur un bord latéral de la partie supérieure de la feuille, et adaptée pour recueillir des fluides lors de l'accouchement, et
- le champ comprend deux poches latérales, s'étendant en regard et fixées sur deux bords latéraux opposés de la partie supérieure de la feuille.

### BREVE DESCRIPTION DES DESSINS

D'autres caractéristiques, buts et avantages de la présente invention apparaîtront mieux à la lecture de la description détaillée qui va suivre, et au regard des dessins annexés donnés à titre d'exemples non limitatifs et sur lesquels :
La figure 1 est une vue schématique du dessus d'un exemple de champ d'accouchement selon l'invention, et
La figure 2 est une vue en perspective d'un exemple de table de travail et d'accouchement pouvant être protégée par un champ selon l'invention.

### DESCRIPTION DETAILLEE D'UN MODE DE REALISATION

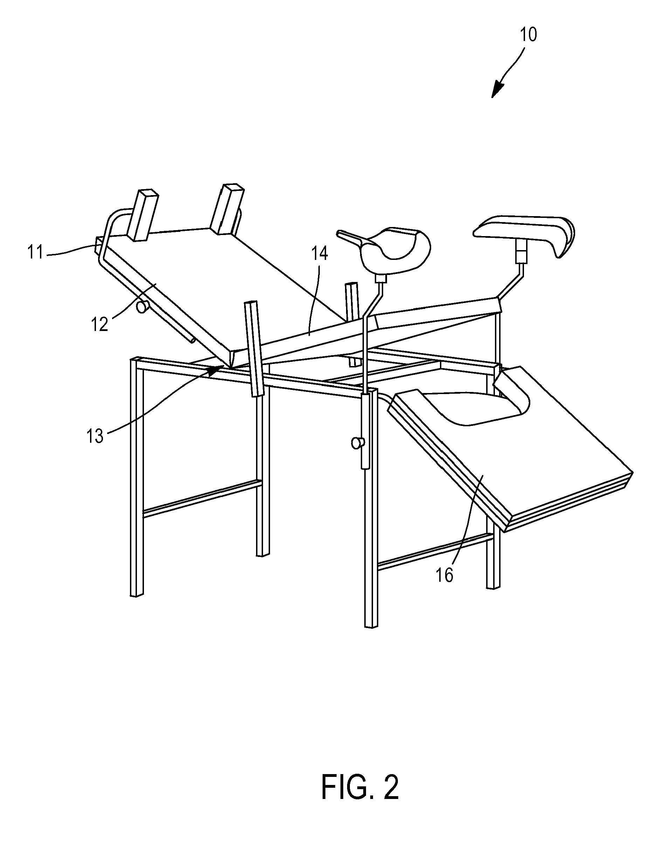

Un exemple de table 10 de travail et d'accouchement a été illustré en figure 2 et comprend un dossier 12, une assise 14 et une partie formant support 16 pour les jambes notamment, s'étendant dans le prolongement de l'assise 14 et pouvant être abaissée ou escamotée sous celle-ci, selon la position choisie par la parturiente. Le dossier 12 et l'assise 14 de la table 10 sont également mobiles entre elles et permettent le passage de la table 10 en position de Trendelenburg. Le dossier 12 et l'assise forment alors un V.

Un champ d'accouchement 1 conforme à l'invention est adapté pour protéger une telle table 10 de travail et d'accouchement tout au long de l'accouchement, c'est-à-dire du travail à la mesure des pertes sanguines, quelle que soit la position d'accouchement choisie.

Pour cela, le champ d'accouchement 1 comprend une feuille 2 souple présentant une partie supérieure 3 destinée à recevoir une parturiente et une partie inférieure 4, s'étendant dans le prolongement de la partie supérieure 3. La partie supérieure 3 de la feuille 2 peut notamment être posée de manière à recouvrir en tout ou partie le dossier 12 et l'assise 14 de la table 10. De préférence, la partie supérieure 3 recouvre la totalité de l'assise 14, la ligne de cassure 13 entre l'assise 14 et le dossier 12, ainsi qu'au moins une partie du dossier 12.

La partie inférieure 4 de la feuille 2 quant à elle s'étend depuis la partie supérieure 3 de la feuille 2, et peut soit reposer en partie sur la partie formant support 16 de la table 10, soit pendre depuis l'assise 14 vers le sol. La partie inférieure 4 est de préférence fixée sur la partie supérieure 3. Le cas échéant, la partie inférieure 4 et la partie supérieure 3 peuvent être formées intégralement et en une seule pièce.

Contrairement aux champs absorbants habituellement utilisés pour l'accouchement, qui sont de faibles dimensions (de l'ordre de 1m sur 1,40m) et ne recouvrent qu'une partie de l'assise 14, le champ d'accouchement 1 selon l'invention recouvre donc en grande partie la table 10 de travail et d'accouchement, ce qui permet de mieux la protéger des fluides physiologiques et d'éliminer le risque d'infection croisée de la parturiente, dans la cas où la table 10 n'aurait pas été correctement nettoyée suite à un accouchement précédent. Par exemple, pour une table 10 conventionnelle, le champ d'accouchement 1 de l'invention peut mesurer entre 2 m et 3 m de longueur pour 80 cm à 1,20 m de largeur, par exemple 2,40 m de longueur pour 90 cm de largeur.

Afin de garantir le bon positionnement du champ d'accouchement 1 sur la table 10 tout au long de l'accouchement et éviter ainsi que la table 10 ne vienne en contact avec des fluides, la feuille 2 peut en outre comprendre des moyens de fixation 5 du champ 1 à la table 10. Le champ d'accouchement 1 est en effet très sollicité au cours de l'accouchement, en raison des changements de position de la table 10 et/ou de la parturiente.

Les moyens de fixation peuvent notamment comprendre un rabat 5, formé au niveau d'un bord supérieur 30 de la partie supérieure 3 de la feuille 2. Ce type de moyen de fixation permet en effet d'adapter facilement le champ d'accouchement 1 à tout type de table 10 du marché. Le rabat 5 peut notamment être obtenu en repliant le bord supérieur 30 de la partie supérieure 3 et en le soudant ou en le collant à l'aide d'un adhésif adapté dans cette position. Il suffit alors d'enfiler le bord supérieur 11 du dossier 12 de la table 10 dans le rabat 5, puis de positionner le reste de la feuille 2 sur la table 10 afin de garantir son positionnement durant toute la durée de l'accouchement.

En variante, les moyens de fixation 5 peuvent également comprendre des bandes adhésives ou un rabat rapporté et fixé sur le bord supérieur 30 de la partie supérieure 3 du champ d'accouchement 1.

Le champ d'accouchement 1 peut en outre comprendre une poche de recueil 6, fixée sur la feuille 2 de manière à s'étendre sous sa partie inférieure 4. Par exemple, la poche de recueil 6 peut être fixée dans une portion inférieure 35 de la partie supérieure 2, la partie inférieure 4 de la feuille 2 recouvrant à la fois la poche de recueil 6 et cette portion 35 de la partie supérieure 3.

La poche de recueil 6 peut être amovible, et comprendre des moyens de fixation configurés pour permettre de la détacher après utilisation. Par exemple, tout ou partie d'une face de la poche 6 peut être fixée sur la feuille 2, par exemple sur la portion inférieure 35, par thermoscellage d'une bande intermédiaire formée d'un multicouche pelable (pouvant comprendre notamment au moins deux couches, dont une basse densité, par exemple une couche en polyéthylène ou en polyéthylène haute densité et une couche en polyéthylène basse ou très basse densité) ou encore au moyen d'adhésifs sur la feuille 2.

Le champ peut en outre comprendre un pagne 8, logé dans la poche de recueil 6. Ce pagne 8 permettra, comme nous le verrons par la suite, de remettre le champ d'accouchement 1 au propre et de permettre à l'opérateur d'effectuer son geste opératoire si cela s'avère nécessaire dans des conditions aseptiques. Selon une forme de réalisation, le pagne 8 peut être fixé au niveau d'un de ses bords sur une face interne de la poche de recueil 6.

La poche 6 peut avoir toute forme appropriée, notamment une forme triangulaire. Elle peut être réalisée dans un film plastique transparent et comprendre, sur une face opposée à la face qui est en regard du champ d'accouchement 1, des graduations permettant de mesurer un volume de fluides remplissant ladite poche 6, tel que les pertes sanguines.

Pour faciliter sa manipulation, la poche 6 peut comporter en outre des poignées, telles que des anses prédécoupées, rapportées et fixées au niveau de son embouchure. Elle peut en outre comprendre des moyens adhésifs pour fermer son embouchure de manière étanche. Par exemple, un adhésif double face protégé le cas échéant par une pellicule pelable peut être appliqué sur une périphérie de son embouchure.

Par ailleurs, la poche 6 peut être simple ou multiple, c'est-à-dire comporter un compartiment unique ou plusieurs compartiments.

La partie inférieure 4 de la feuille 2 peut être amovible, de manière à permettre à un opérateur de dévoiler la poche de recueil 6 et de remettre le champ 1 au propre pour la mesure des pertes sanguines. Pour cela, la partie inférieure 4 peut être fixée sur un bord inférieur 31 de la partie supérieure 3 par l'intermédiaire d'un adhésif approprié, ou par thermoscellage d'une bande intermédiaire formée d'un multicouche pelable comme décrit ci-dessus. En variante, la partie inférieure 4 et la partie supérieure 3 peuvent former une seule feuille 2 présentant une ligne de faiblesse, qui peut être un amincissement local selon l'épaisseur de la feuille 2 ou une ligne prédécoupée.

Selon une autre variante encore, la partie inférieure 4 de la feuille 2 peut être formée en une seule pièce avec sa partie supérieure 3, sans ligne de faiblesse. L'opérateur peut alors découper ou déchirer la partie inférieure 4 afin d'accéder à la poche de recueil 6 qui est sous-jacente.

La feuille 2 du champ d'accouchement 1 est étanche et forme une barrière aux fluides, afin de préserver la table 10 de travail et d'accouchement. A cette fin, la feuille 2 peut par exemple être réalisée en polyéthylène.

La partie inférieure 4 peut être doublée et comprendre une face supérieure 40 comprenant un matériau absorbant en vue d'absorber les fluides physiologiques au cours de l'accouchement. Pour cela, la partie inférieure 4 peut par exemple comprendre une couche inférieure étanche 42 afin d'empêcher que les fluides ne traversent la feuille 2, recouverte d'une couche absorbante 40. La couche absorbante 40 peut notamment être collée sur la couche inférieure 42 étanche. Par exemple, la couche inférieure 42 peut être en polyéthylène, tandis que la couche absorbante 40 peut être de type Absorbex®.

Dans une forme de réalisation, la face supérieure 34, 36 de la partie supérieure 3 est hydrophobe, afin de faciliter l'évacuation des fluides physiologiques, notamment lorsqu'ils se concentrent dans la ligne de cassure 13 entre le dossier 12 et l'assise 14 de la table 10. Par exemple, la partie supérieure 3 peut comprendre uniquement une couche étanche 34, par exemple en polyéthylène, ou être doublée d'une couche 36 en nontissé hydrophobe afin d'améliorer le confort de la parturiente. La couche 36 de nontissé hydrophobe peut par exemple être collée directement sur la couche étanche 34. Contrairement à l'enseignement du document US 2005/022822, la face supérieure 34, 36 du champ d'accouchement 1 est donc hydrophobe, et non absorbante.

On notera par ailleurs que la protection de la table 10 de travail et d'accouchement est encore améliorée lorsque la partie supérieure 3 comprend une couche étanche 34 doublée d'une couche 36 en nontissé hydrophobe : en effet, il s'avère à l'usage que la mise en oeuvre d'une partie supérieure 3 ne comprenant qu'une couche de nontissé hydrophobe, par exemple du type SMMS en polypropylène, ne suffit pas pour protéger la table étant données les pressions appliquées en présence de fluides par la parturiente au cours de l'accouchement, tandis qu'une partie supérieure 3 ne comprenant qu'une couche étanche de type polyéthylène ne confère pas un confort suffisant à la parturiente.

Avantageusement, la face de la feuille 2 sur laquelle sont collées la couche 36 de nontissé hydrophobe et/ou la couche absorbante 40 correspondent à la face destinée à venir en contact avec la parturiente lors de l'accouchement, les faces 42 et 34 étant disposées face à la table 10.

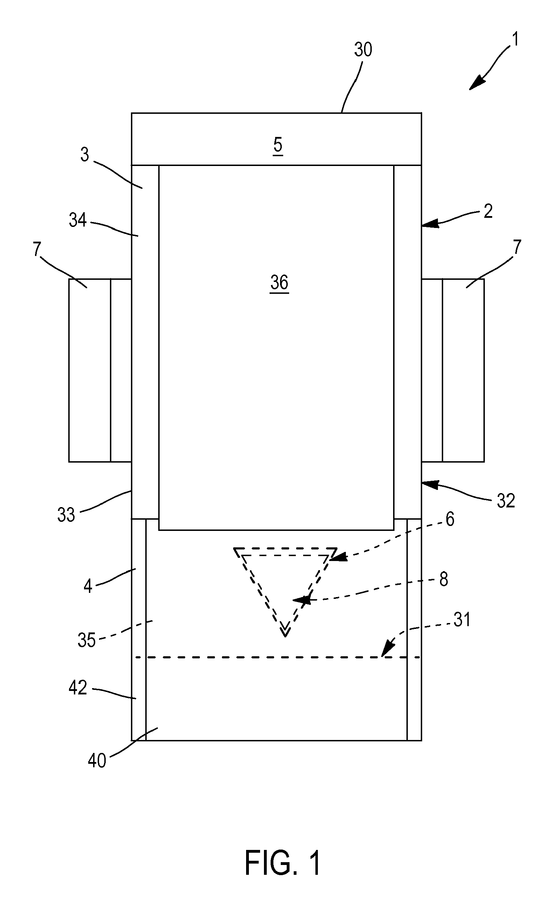

Selon une forme de réalisation, le champ d'accouchement 1 peut en outre comprendre une ou plusieurs poches latérales 7, fixées sur ses bords latéraux 32, 33 afin de récupérer les fluides physiologiques. Ainsi, dans l'exemple de réalisation illustré sur la figure 1, le champ d'accouchement 1 comprend une première poche latérale 7, fixée sur un premier bord latéral 32 de la feuille 2, et une deuxième poche latérale 7, fixée sur le bord latéral 33 en regard de la feuille 2. Ces poches latérales 7 peuvent être agencées sur la partie supérieure 3 de la feuille 2 de manière à venir en regard de la ligne de cassure 13 entre le dossier 12 et l'assise 14, afin de recueillir les fluides physiologiques qui s'y concentrent lorsque la table 10 de travail et d'accouchement est placée dans la position de Trendelenburg.

Dans le cas où la partie supérieure 3 est hydrophobe, les flux physiologiques sont alors redirigés par la ligne de cassure 13 de la table 10 directement dans les poches latérales 7, ce qui améliore la protection de la table 10.

Les poches latérales 7 peuvent être détachables, afin d'être évacuées suite à la délivrance.

Dans une variante de réalisation, le champ d'accouchement 1 peut en outre comprendre des moyens d'attache, fixés dans la partie inférieure 4, adaptés pour venir s'attacher sur des vêtements d'un opérateur en cas d'expulsion non prévue du bébé, afin de fournir une protection minimale à l'opérateur si celui-ci ne dispose pas de suffisamment de temps pour enfiler une casaque. Les moyens d'attache peuvent notamment comprendre un adhésif ou une bande de velours-crochets.

Nous allons à présent décrire un exemple d'utilisation du champ d'accouchement 1 dans le cadre d'un accouchement.

Dans un premier temps, lorsque le travail commence pour la parturiente, le champ d'accouchement 1 est placé sur une table 10 de travail et d'accouchement. Lorsque le champ d'accouchement 1 comprend des moyens de fixation 5, l'opérateur peut fixer le champ 1 sur la table 10.

Dans la suite de la description sera décrite la forme de réalisation dans laquelle le champ d'accouchement 1 comprend deux poches latérales 7, fixées sur la partie supérieure 3, un rabat 5 et une poche de recueil 6 fixée sur la feuille de manière à être cachée par la partie inférieure 4. Ceci n'est cependant pas limitatif, le champ d'accouchement 1 pouvant ne comprendre ni rabat 5, ni poche latérale 7 (ou un nombre différent de poches latérales 7), et que la poche de recueil 6 peut être rapportée sur le champ d'accouchement 1.

Par exemple, dans le mode de réalisation illustré en figure 1 où le champ d'accouchement 1 comprend un rabat 5, l'opérateur peut enfiler le rabat 5 sur le bord supérieur 11 du dossier 12 de la table 10 et installer la feuille 2 de manière à ce que celle-ci recouvre le dossier 12, l'assise 14 et le cas échéant la partie support 16 de la table 10. Le champ d'accouchement 1 est donc à présent en position afin d'accueillir la parturiente et est capable de protéger tout ou partie du dossier 12 et l'assise 14 de la table 10 de travail et d'accouchement malgré les différents mouvements de la table 10 et/ou de la parturiente durant l'accouchement.

Avant utilisation, le champ d'accouchement 1 peut être emballé de manière à ce que le rabat 5 soit accessible en premier, le reste de la feuille 2 et la poche de recueil 6 pouvant ensuite être déballés en déroulant simplement la feuille 2 depuis le rabat 5 sur la table 10.

En installant le champ d'accouchement 1 sur la table 10, l'opérateur veille à positionner la feuille 2 de sorte que les poches latérales 7 viennent en regard de la ligne de cassure 13 de la table 10 afin que celles-ci puissent récupérer les fluides provenant de l'accouchement. Le cas échéant, l'opérateur peut ouvrir les poches latérales 7 et les maintenir en position ouverte à l'aide de moyens connus.

La parturiente peut alors s'allonger sur la table 10 ainsi protégée par le champ d'accouchement 1. La table 10 peut ensuite être mise dans la position adaptée au stade du travail de la patiente, qui correspond généralement à la position de Trendelenburg.

Tout au long du travail de la parturiente, aucun champ supplémentaire n'est nécessaire, le champ d'accouchement 1 selon l'invention protégeant la table 10 et suivant son déplacement, malgré les mouvements de la parturiente. Par ailleurs, grâce au caractère hydrophobe de la partie supérieure 3, tous les fluides physiologiques sont évacués vers les poches latérales 7, protégeant ainsi la table 10 de travail et d'accouchement, ainsi que la salle dans laquelle a lieu l'accouchement.

Lorsque la délivrance approche, la table 10 est mise en position gynécologique (ou toute autre position choisie). La partie support 16 de la table 10 est alors repliée de 90° ou escamotée sous l'assise 14.

Après la délivrance, la partie inférieure 4 du champ d'accouchement, qui a absorbé une partie des fluides physiologiques, 1 peut être détachée de la partie supérieure 3 afin de dévoiler la poche de recueil 6. Pour cela, la partie inférieure 4 est soit pelée, soit déchirée le long de la ligne de faiblesse, soit découpée, selon le mode de réalisation du champ 1. Cette partie inférieure 4 du champ d'accouchement 1 peut alors être évacuée.

La poche de recueil 6, qui est fixée ici dans la portion inférieure 35 de la partie supérieure 3, peut alors recueillir les pertes sanguines. Le pagne 8 est en outre sorti de la poche de recueil 6 pour être positionné sous les fesses de la parturiente afin de remettre cette zone du champ d'accouchement 1 au propre, d'anticiper tout risque d'hémorragie de la parturiente et permettre à l'opérateur d'effectuer, si cela est nécessaire, la suture d'épisiotomie.

Le pagne 8 permet en outre de diriger les pertes sanguines vers la poche de recueil 6. De la sorte, le champ d'accouchement 1 de l'invention permet la quantification des pertes sanguine sans besoin de champ supplémentaire dédié.

On notera que le pagne 8 et la poche de recueil 6 ayant été protégés pendant tout le travail et la délivrance par la partie inférieure 4 du champ d'accouchement 1, sont stériles. Le cas échéant, l'opérateur peut donc effectuer des gestes opératoires dans des conditions d'aseptie stériles, bien que l'accouchement ait lieu dans un milieu non stérile.

Une fois l'accouchement et l'éventuelle suture d'épisiotomie terminés, l'opérateur peut alors évacuer le champ d'accouchement 1, la poche de recueil 6 et les poches latérales 7.

Le temps de nettoyage et les risques d'endommagement de la table 10 de travail et d'accouchement sont fortement réduits grâce au champ d'accouchement 1 de l'invention.

## Revendications

1. Champ d'accouchement (1), comprenant une feuille (2) souple, ladite feuille (2) souple présentant une partie supérieure (3), destinée à recevoir une parturiente, et une partie inférieure (4), s'étendant dans le prolongement de ladite partie supérieure (3),
le champ d'accouchement (1) étant **caractérisé en ce que** la partie supérieure (3) comprend une face supérieure (34, 36) qui est destinée à venir en contact avec la parturiente, ladite face supérieure (34, 36) étant réalisée dans un matériau hydrophobe formant barrière aux liquides, et **en ce que** la partie inférieure (4) de la feuille (2) comprend une face supérieure (40) comprenant un matériau absorbant.

2. Champ d'accouchement (1) selon la revendication 1, dans lequel la partie inférieure (4) de la feuille (2) est fixée de manière amovible sur la partie supérieure (3).

3. Champ d'accouchement (1) selon la revendication 2, dans lequel la partie inférieure (4) est fixée sur un bord inférieur (31) de la partie supérieure (3) par l'intermédiaire d'un adhésif approprié ou par thermoscellage d'une bande intermédiaire formée d'un multicouche pelable, ou est reliée à la partie supérieure (3) par l'intermédiaire d'une ligne de faiblesse.

4. Champ d'accouchement selon l'une des revendications 1 à 3, comprenant en outre une poche de recueil (6), fixée sur la feuille (2) de manière à s'étendre sous la partie inférieure (4) et à être protégée par ladite partie inférieure au cours de l'accouchement.

5. Champ d'accouchement (1) selon la revendication 4, dans lequel la poche de recueil (6) est fixée dans une portion inférieure (35) de la partie supérieure de la feuille (2) supérieure.

6. Champ d'accouchement (1) selon l'une des revendications 4 ou 5, comprenant en outre un pagne (8) logé dans la poche de recueil (6), ledit pagne (8) pouvant être fixé sur une face interne de ladite poche de recueil (6).

7. Champ d'accouchement (1) selon l'une des revendications 1 à 6, dans lequel la feuille (2) est réalisée dans un matériau hydrophobe formant barrière aux liquides du type polyéthylène.

8. Champ d'accouchement (1) selon la revendication 7, dans lequel le matériau absorbant de la face supérieure (40) de la partie inférieure (4) est fixé sur ladite feuille (2).

9. Champ d'accouchement (1) selon l'une des revendications 1 à 8, dans lequel la face supérieure (36) de la partie supérieure (3) comprend un nontissé hydrophobe.

10. Champ d'accouchement (1) selon l'une des revendications 1 à 9 adapté pour être placé sur une table (10) de travail et d'accouchement, dans lequel la feuille (2) comprend en outre des moyens de fixation (5) à ladite table (10) d'accouchement.

11. Champ d'accouchement (1) selon la revendication 10, dans lequel les moyens de fixation comprennent un rabat (5), formé dans une partie supérieure (3) de la feuille (2), adapté pour être enfilé sur un bord supérieur (11) de la table (10) de travail et d'accouchement.

12. Champ d'accouchement (1) selon l'une des revendications 1 à 11, comprenant en outre au moins une poche latérale (7), fixée sur un bord latéral (32, 33) de la partie supérieure (3) de la feuille (2), et adaptée pour recueillir des fluides lors de l'accouchement.

13. Champ d'accouchement (1) selon la revendication 12, comprenant deux poches latérales (7), s'étendant en regard et fixées sur deux bords latéraux (32, 33) opposés de la partie supérieure (3) de la feuille (2).

14. Champ d'accouchement (1) selon l'une des revendications 1 à 13, dans lequel la feuille (2) présente une longueur comprise entre deux mètres et trois mètres.
